# EUROPEAN PATENT APPLICATION

(11) **EP 2 325 166 A1**
(43) Date of publication of application: **25.05.2011**
(21) Application number: 09805023.0
(22) Date of filing: 06.08.2009
(51) Int. Cl.: C07C 263/18, C07C 263/10, C07C 263/16, C07C 265/04

(54) **METHOD FOR INHIBITING POLYMERIZATION OF ETHYLENICALLY UNSATURATED CARBOXYLIC ACID ESTER COMPOUND CONTAINING ISOCYANATE GROUP AND METHOD FOR PRODUCING ETHYLENICALLY UNSATURATED CARBOXYLIC ACID ESTER COMPOUND CONTAINING ISOCYANATE GROUP**

(30) Priority: 08.08.2008 JP 2008205738
(71) Applicant: Showa Denko K.K., Tokyo 105-8518 (JP)
(72) Inventor: NISHIMURA, Norihito, Aizuwakamatsu-shi Fukushima 969-3431 (JP); YOROZUYA, Shinichi, Aizuwakamatsu-shi Fukushima 969-3431 (JP)
(74) Representative: Strehl Schübel-Hopf & Partner
(86) International application number: PCT/JP2009/063932
(87) International publication number: WO 2010/016540

(57) **Abstract**

It is an object of the invention to provide a process of inhibiting polymerization of isocyanate group-containing, ethylenically unsaturated carboxylate compounds, and a process for producing isocyanate group-containing, ethylenically unsaturated carboxylate compounds which involves the polymerization inhibition process.

The process of inhibiting polymerization of isocyanate group-containing, ethylenically unsaturated carboxylate compounds of the invention includes controlling the oxygen gas (O₂) concentration in a liquid phase containing an isocyanate group-containing, ethylenically unsaturated carboxylate compound to not less than 0.5 mg/L. In a preferred embodiment, a gas containing oxygen gas (O₂) is introduced to the liquid phase. In another preferred embodiment, the oxygen gas (O₂) concentration in the liquid phase is measured with an electrode that is immersed in the liquid phase.

## Description

### TECHNICAL FIELD

The present invention relates to a process of inhibiting polymerization of isocyanate group-containing, ethylenically unsaturated carboxylate compounds, and to a process of producing the compounds. In detail, the invention relates to a process which inhibits the polymerization of isocyanate group-containing, ethylenically unsaturated carboxylate compounds by means of an oxygen gas (O₂) present in a liquid phase containing the isocyanate group-containing, ethylenically unsaturated carboxylate compounds, and to a process of producing isocyanate group-containing, ethylenically unsaturated carboxylate compounds which involves the polymerization inhibition process.

### BACKGROUND ART

Ethylenically unsaturated compounds are highly liable to be polymerized by heat, light and other factors. Because of this tendency, the compounds are often polymerized during the production steps and purification steps to form foreign matters such as polymers or oligomers, which cause troubles in the production facilities. The attachment of the foreign matters to production tanks, distillation columns and pipes in the production steps and purification steps for ethylenically unsaturated compounds not only leads to blockage or sticking of movable equipment but also causes troubles in the production and purification of the ethylenically unsaturated compounds. The removal of foreign matters attached in the related facilities relies on manual procedures and is thus not efficient, and the facilities have to be shutdown for a long term causing great economic loss. Further, such foreign matters can lower the quality of the products.

Meanwhile, it is known that molecular oxygen is effective in inhibiting the polymerization of ethylenically unsaturated compounds. A large number of methods have been proposed and carried out wherein a molecular oxygen gas is supplied and controlled to inhibit polymerization of ethylenically unsaturated compounds and thereby to prevent the occurrence of foreign matters in production facilities for the compounds.

For example, Patent Literature 1 discloses a production process in which the oxygen concentration in the gas phase is measured and controlled. This process does not directly control the oxygen concentration in the liquid phase which is an actual target of the polymerization inhibition. Accordingly, gas-liquid equilibrium is not reached and the process cannot detect whether sufficient oxygen is present in the liquid phase. Patent Literature 2 discloses a production process in which the oxygen concentration in the system is controlled. The patent literature describes that a portion of the liquid phase is withdrawn from the production apparatus and the oxygen concentration therein is analyzed. However, because the gas-liquid equilibrium is disturbed by the withdrawal and the patent literature does not disclose any analysis methods, it is uncertain whether the oxygen concentration in the liquid phase is measured accurately.

Under the above circumstances, there has been a strong demand for effective processes of preventing polymerization of ethylenically unsaturated compounds.

### Citation List

### Patent Literatures

Patent Literature 1: JP-A-H09-67311
Patent Literature 2: JP-A-2007-284355

### SUMMARY OF INVENTION

### Technical Problem

It is an object of the present invention to provide a process of inhibiting polymerization of isocyanate group-containing, ethylenically unsaturated carboxylate compounds, and a process for producing isocyanate group-containing, ethylenically unsaturated carboxylate compounds which involves the polymerization inhibition process.

### Solution to Problem

The present inventors studied in detail the polymerization characteristics of isocyanate group-containing, ethylenically unsaturated carboxylate compounds. They have then found that controlling the oxygen gas (O₂) concentration in a liquid phase is effective in inhibiting the polymerization of isocyanate group-containing, ethylenically unsaturated carboxylate compounds. The present invention has been completed based on the finding. For example, the present invention is concerned with the following [1] to [14].
[1] A process of inhibiting polymerization of isocyanate group-containing, ethylenically unsaturated carboxylate compounds, which comprises controlling the oxygen gas (O₂) concentration in a liquid phase containing an isocyanate group-containing, ethylenically unsaturated carboxylate compound to not less than 0.5 mg/L.
[2] The process of inhibiting polymerization of isocyanate group-containing, ethylenically unsaturated carboxylate compounds as described in [1], which comprises introducing a gas containing oxygen gas (O₂) to the liquid phase.
[3] The process of inhibiting polymerization of isocyanate group-containing, ethylenically unsaturated carboxylate compounds as described in [2], wherein the gas containing oxygen gas (O₂) is air, diluted air or diluted oxygen.
[4] The process of inhibiting polymerization of isocyanate group-containing, ethylenically unsaturated carboxylate compounds as described in [2] or [3], wherein the process comprises:
   a step of measuring the oxygen gas (O₂) concentration in the liquid phase; and
   a step of controlling the supply amount of oxygen gas (O₂) by controlling the introduction of the gas containing oxygen gas (O₂), based on the result of the measurement.
[5] The process of inhibiting polymerization of isocyanate group-containing, ethylenically unsaturated carboxylate compounds as described in any one of [1] to [4], wherein the oxygen gas (O₂) concentration in the liquid phase is measured with an electrode that is immersed in the liquid phase.
[6] The process of inhibiting polymerization of isocyanate group-containing, ethylenically unsaturated carboxylate compounds as described in any one of [1] to [5], wherein the liquid phase is a bottom liquid in a distillation step.
[7] The process of inhibiting polymerization of isocyanate group-containing, ethylenically unsaturated carboxylate compounds as described in any one of [1] to [6], wherein the isocyanate group-containing, ethylenically unsaturated carboxylate compound is a compound represented by Formula (A) or (B) below:

   CH₂=CR⁵-COO-R⁶-NCO (A)

   wherein R⁵ is a hydrogen atom or a methyl group, and R⁶ is a C1-10, optionally branched alkylene group, a hydrocarbon group wherein a C3-6 cycloalkylene group has C0-3 alkylene groups at front and back ends thereof, or a C6-8 aromatic hydrocarbon ring;

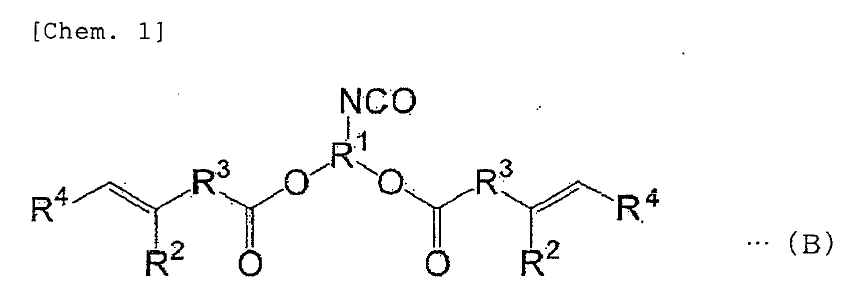

wherein R¹ is a C1-10, linear or branched saturated aliphatic group, each R² is independently a hydrogen atom or a methyl group, each R³ is independently a C0-5, linear or branched alkylene group, and each R⁴ is independently a hydrogen atom, a C1-6, linear or branched alkyl group, or an aryl group.
[8] The process of inhibiting polymerization of isocyanate group-containing, ethylenically unsaturated carboxylate compounds as described in [7], wherein the isocyanate group-containing, ethylenically unsaturated carboxylate compound is 2-methacryloyloxyethyl isocyanate, 2-acryloyloxyethyl isocyanate, 1,1-bis(acryloyloxymethyl)ethyl isocyanate, 2-(isocyanatoethyloxy)ethyl methacrylate or 2-(isocyanatoethyloxy)ethyl acrylate.
[9] The process of inhibiting polymerization of isocyanate group-containing, ethylenically unsaturated carboxylate compounds as described in any one of [1] to [8], wherein the water content in the liquid phase is not more than 5% by mass.
[10] A process for producing isocyanate group-containing, ethylenically unsaturated carboxylate compounds, comprising performing the process of inhibiting polymerization described in any one of [1] to [9].
[11] A process for producing isocyanate group-containing, ethylenically unsaturated carboxylate compounds represented by Formula (A), the process comprising:
   a step of dehydrochlorinating an isocyanate group-containing, 3-chloropropionate derivative represented by Formula (a1) in the presence of a basic nitrogen compound; and
   a step of controlling the oxygen gas (O₂) concentration in a liquid phase to not less than 0.5 mg/L;

      Cl-CH₂-CHR⁵-COO-R⁶-NCO (a1)

      CH₂=CR⁵-COO-R⁶-NCO (A)

      wherein R⁵ is a hydrogen atom or a methyl group, and R⁶ is a C1-10, optionally branched alkylene group, a hydrocarbon group wherein a C3-6 cycloalkylene group has C0-3 alkylene groups at front and back ends thereof, or a C6-8 aromatic hydrocarbon ring.
[12] A process for producing isocyanate group-containing, ethylenically unsaturated carboxylate compounds represented by Formula (B), the process comprising:
   a step of dehydrochlorinating an isocyanate compound represented by Formula (b1) in the presence of a basic nitrogen compound; and
   a step of controlling the oxygen gas (O₂) concentration in a liquid phase to not less than 0.5 mg/L;

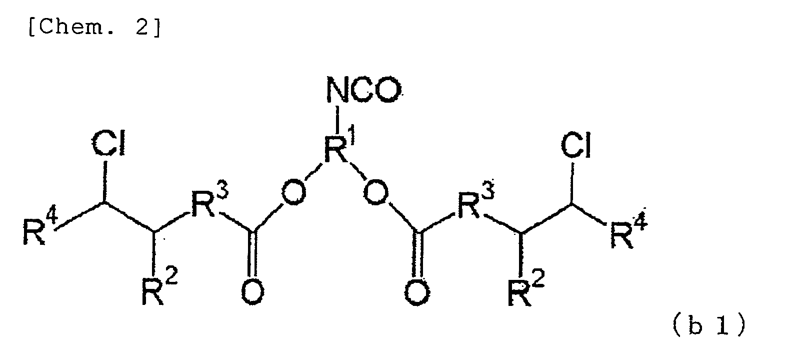

wherein R¹ is a C1-10, linear or branched saturated aliphatic group, each R² is independently a hydrogen atom or a methyl group, each R³ is independently a C0-5, linear or branched alkylene group, and each R⁴ is independently a hydrogen atom, a C1-6, linear or branched alkyl group, or an aryl group;

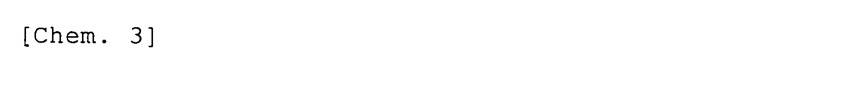

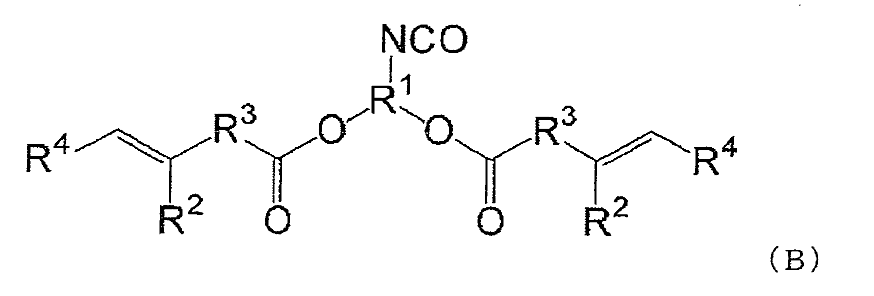

wherein R¹ to R⁴ are the same as above.
[13] A process for producing isocyanate group-containing, ethylenically unsaturated carboxylate compounds, the process comprising:
   a step of obtaining an ester compound of Formula (b4) from a dihydroxyamine mineral acid salt compound of Formula (b2) and a compound of Formula (b3);
   a step of obtaining an isocyanate group-containing, ethylenically unsaturated carboxylate compound of Formula (B') from the ester compound of Formula (b4) and a compound of Formula (b5) ; and
   a step of controlling the oxygen gas (O₂) concentration in a liquid phase to not less than 0.5 mg/L;

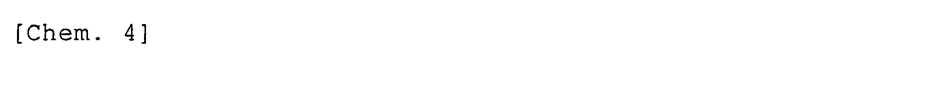

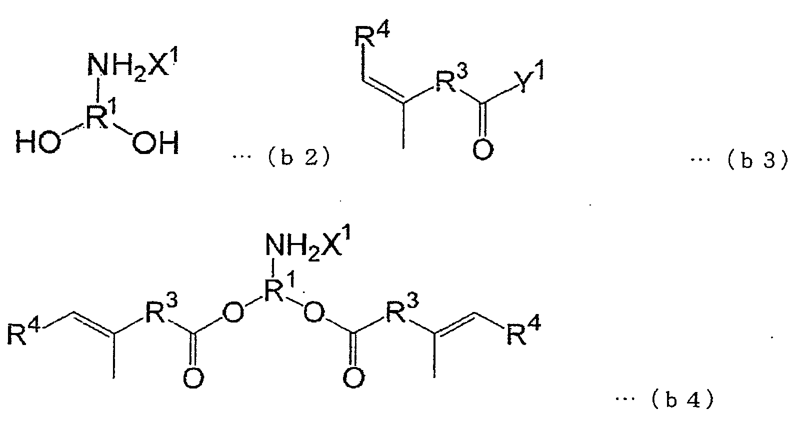

wherein R¹ is a C1-10, linear or branched saturated aliphatic group, X¹ is a mineral acid, each R³ is independently a C0-5, linear or branched alkylene group, each R⁴ is independently a hydrogen atom, a C1-6, linear or branched alkyl group, or an aryl group, and Y¹ is a hydroxyl group, a chlorine atom or R⁶O- (wherein R⁶ is a C1-6 alkyl group);

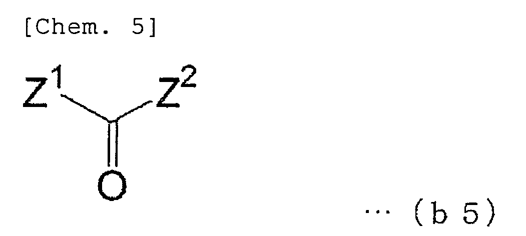

wherein Z¹ and Z² are each independently a chlorine atom, a bromine atom, R⁷O- (wherein R⁷ is a C1-6, linear or branched alkyl group, a C1-6, linear or branched alkenyl group or an optionally substituted aryl group), an imidazole residue or a pyrazole residue;

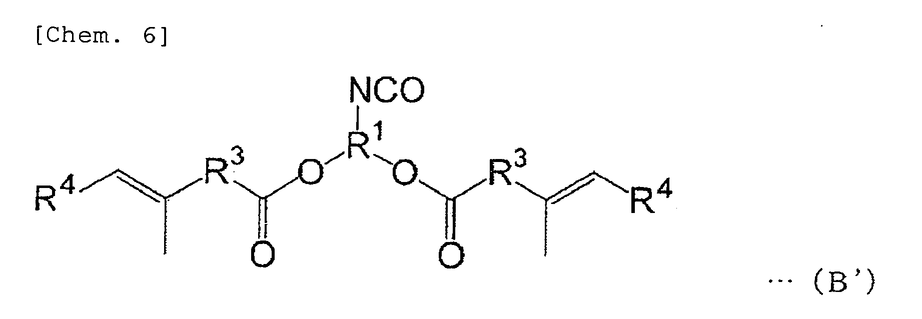

wherein R¹, R³ and R⁴ are the same as above.
[14] The process for producing isocyanate group-containing, ethylenically unsaturated carboxylate compounds described in any one of [11] to [13], wherein the step of controlling the oxygen gas (O₂) concentration in a liquid phase to not less than 0.5 mg/L comprises:
   a step of measuring the oxygen gas (O₂) concentration in the liquid phase;
   a step of controlling the supply amount of oxygen gas (O₂) by controlling the introduction of a gas containing oxygen gas (O₂), based on the result of the measurement; and
   a step of introducing the gas containing oxygen gas (O₂) to the liquid phase.

### Advantageous Effects of Invention

According to the present invention, the polymerization of isocyanate group-containing, ethylenically unsaturated carboxylate compounds is inhibited by controlling a liquid phase containing an isocyanate group-containing, ethylenically unsaturated carboxylate compound to a specific oxygen gas (O₂) concentration. In detail, the oxygen gas (O₂) concentration in the liquid is accurately measured and the amount of oxygen gas (O₂) supplied to the liquid phase is controlled based on the measurement result to maintain the oxygen gas (O₂) concentration in the liquid phase to a desired range, whereby the polymerization of isocyanate group-containing, ethylenically unsaturated carboxylate compounds is inhibited effectively. By involving the polymerization inhibition process, isocyanate group-containing, ethylenically unsaturated carboxylate compounds may be manufactured efficiently.

### DESCRIPTION OF EMBODIMENTS

The present invention will be described in detail hereinbelow.

In the specification, the words "isocyanate group-containing, ethylenically unsaturated carboxylate compounds" refer to carboxylate compounds having an ethylenically unsaturated group and an isocyanate group in the molecule. Such compounds will be hereinafter also referred to as the "isocyanate group-containing, ethylenically unsaturated compounds". The word "(meth)acryloyl" refers to acryloyl or methacryloyl, part of the hydrogen atoms of which may be substituted.

In the process of inhibiting polymerization of isocyanate group-containing, ethylenically unsaturated carboxylate compounds according to the present invention, the oxygen gas (O₂) concentration in a liquid phase containing an isocyanate group-containing, ethylenically unsaturated carboxylate compound is controlled to a specific range.

### (Isocyanate group-containing, ethylenically unsaturated carboxylate compounds)

The isocyanate group-containing, ethylenically unsaturated carboxylate compounds in the invention are not particularly limited as long as the isocyanate group-containing, ethylenically unsaturated carboxylate compounds are liquid around room temperature. Examples thereof include compounds represented by Formula (A) or (B) below:

CH₂=CR⁵-COO-R⁶-NCO (A)

wherein R⁵ is a hydrogen atom or a methyl group, and R⁶ is a C1-10, optionally branched alkylene group, a hydrocarbon group wherein a C3-6 cycloalkylene group has C0-3 alkylene groups at front and back ends thereof, or a C6-8 aromatic hydrocarbon ring;

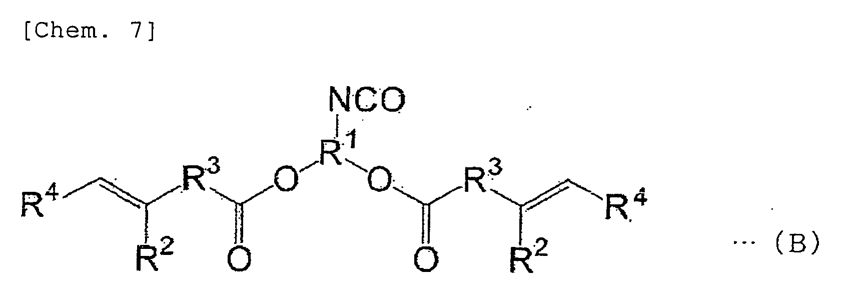

wherein R¹ is a C1-10, linear or branched saturated aliphatic group, each R² is independently a hydrogen atom or a methyl group, each R³ is independently a C0-5, linear or branched alkylene group, and each R⁴ is independently a hydrogen atom, a C1-6, linear or branched alkyl group, or an aryl group.
Specific examples of the isocyanate group-containing, ethylenically unsaturated carboxylate compounds include 2-methacryloyloxyethyl isocyanate, 3-methacryloyloxy-n-propyl isocyanate, 2-methacryloyloxy-isopropyl isocyanate, 4-methacryloyloxy-n-butyl isocyanate, 2-methacryloyloxy-tert-butyl isocyanate, 2-methacryloyloxybutyl-4-isocyanate, 2-methacryloyloxybutyl-3-isocyanate, 2-methacryloyloxybutyl-2-isocyanate, 2-methacryloyloxybutyl-1-isocyanate, 5-methacryloyloxy-n-pentyl isocyanate, 6-methacryloyloxy-n-hexyl isocyanate, 7-methacryloyloxy-n-heptyl isocyanate, 2-(isocyanatoethyloxy)ethyl methacrylate, 3-methacryloyloxyphenyl isocyanate, 4-methacryloyloxyphenyl isocyanate, 2-acryloyloxyethyl isocyanate, 3-acryloyloxy-n-propyl isocyanate, 2-acryloyloxy-isopropyl isocyanate, 4-acryloyloxy-n-butyl isocyanate, 2-acryloyloxy-tert-butyl isocyanate, 2-acryloyloxybutyl-4-isocyanate, 2-acryloyloxybutyl-3-isocyanate, 2-acryloyloxybutyl-2-isocyanate, 2-acryloyloxybutyl-1-isocyanate, 5-acryloyloxy-n-pentyl isocyanate, 6-acryloyloxy-n-hexyl isocyanate, 7-acryloyloxy-n-heptyl isocyanate, 2-(isocyanatoethyloxy)ethyl acrylate, 3-acryloyloxyphenyl isocyanate, 4-acryloyloxyphenyl isocyanate, 1,1-bis(methacryloyloxymethyl)methyl isocyanate, 1,1-bis(methacryloyloxymethyl)ethyl isocyanate, 1,1-bis(acryloyloxymethyl)methyl isocyanate, 1,1-bis(acryloyloxymethyl)ethyl isocyanate, and compounds corresponding to the above compounds except that an alkyl hydrogen atom is replaced with fluorine.

Of these, 2-methacryloyloxyethyl isocyanate, 4-methacryloyloxy-n-butyl isocyanate, 5-methacryloyloxy-n-pentyl isocyanate, 6-methacryloyloxy-n-hexyl isocyanate, 2-acryloyloxyethyl isocyanate, 3-methacryloyloxyphenyl isocyanate, 4-methacryloyloxyphenyl isocyanate, 1,1-bis(methacryloyloxymethyl)ethyl isocyanate, 2-(isocyanatoethyloxy)ethyl methacrylate and 2-(isocyanatoethyloxy)ethyl acrylate are preferred.

### (Other components)

In the invention, the liquid phase containing the isocyanate group-containing, ethylenically unsaturated carboxylate compound, or the liquid phase in the production of the compound may contain optional components such as solvent and polymerization inhibitor. Examples of the polymerization inhibitors include phenolic polymerization inhibitors such as BHT (2,6-di-tertiary butyl-p-cresol) and quinone, sulfur polymerization inhibitors such as phenothiazine, phosphorus polymerization inhibitors, and N-oxyl compounds such as TEMPO (2,2,6,6-tetramethylpiperidin-1-oxyl).

### 〈Polymerization〉

In the invention, the term "polymerization" includes the formation of polymers such as formation of solids and solidification (vitrification, gelation) of the liquid phase, and also includes polymerization (for example, oligomerization) of the isocyanate group-containing, ethylenically unsaturated compounds that does not form solids. The occurrence of such polymerization may be confirmed by quantifying the reduction of the isocyanate group-containing, ethylenically unsaturated compounds or determining the amount of oligomers formed, by conventional analytical methods (such as gas chromatography, high-performance liquid chromatography and gel permeation chromatography).

### (Residual rate/reduction rate of isocyanate group-containing, ethylenically unsaturated compound monomers)

The reduction rate of the isocyanate group-containing, ethylenically unsaturated compounds indicates the rate at which the isocyanate group-containing, ethylenically unsaturated compound as a monomer decreases due to the polymerization by heat, light or the like per unit time, relative to the amount of the isocyanate group-containing, ethylenically unsaturated compound at a given previous time at 100%.

The residual rate of the isocyanate group-containing, ethylenically unsaturated compounds may be derived from the equation: Residual rate (%) = 100 (%) - reduction rate (%). From the viewpoint of inhibiting the polymerization of the isocyanate group-containing, ethylenically unsaturated compounds, it is preferable that the residual rate is higher (closer to 100%) and the reduction rate is lower (closer to 0%). Desired levels of the residual rate/the reduction rate vary depending on the temperature, the amount of polymerization inhibitors or the amount of impurities. For example, under conditions where the isocyanate group-containing, ethylenically unsaturated compounds as products are stored with light shielding at around room temperature, the reduction rate is desirably not more than 10%, preferably not more than 5%, and more preferably not more than 2% per year. The reduction rate in the production of the isocyanate group-containing, ethylenically unsaturated compounds is preferably low from the economic viewpoint or the operation control viewpoint, and is for example desirably not more than 1%, preferably not more than 0.5%, and more preferably not more than 0.3% per hour.

### (Water content in liquid phase)

The liquid phase is mainly composed of organic compounds including the isocyanate group-containing, ethylenically unsaturated carboxylate compound and an organic solvent. The water content in the liquid phase is preferably lower, preferably not more than 5% by mass, and more preferably not more than 2% by mass.

### (Oxygen gas (O₂)〉

In the invention, any gases containing oxygen gas (O₂), for example air, may be used without limitation. In view of influences to the reaction or storage, it is preferable to use a dry gas, for example dry air or dry oxygen. Diluted air obtained by diluting dry air with dry nitrogen or the like, or diluted oxygen obtained by diluting dry oxygen with dry nitrogen or the like may be used.

### (Range of oxygen gas (O₂) concentration in liquid phase)

Molecular oxygen dissolved in the liquid phase is consumed by the isocyanate group-containing, ethylenically unsaturated compound when it exhibits polymerization inhibition effects. The oxygen concentration is theoretically sufficient when the amount of molecular oxygen newly dissolved in the liquid phase is larger than the amount of molecular oxygen consumed. It is however necessary that the control value of the concentration of dissolved oxygen gas (O₂) is determined.

A higher concentration of oxygen gas (O₂) in the liquid phase is more preferable. However, the oxygen gas has a saturation concentration and therefore it is difficult to control the oxygen concentration above the saturation concentration. According to gas-liquid equilibrium and the Henry's law, the saturation concentration varies depending on the oxygen partial pressure and the kind of the isocyanate group-containing, ethylenically unsaturated compound. In general organic compounds, the saturation concentration of dry air (containing 21% by volume of oxygen gas (O₂)) blown at atmospheric pressure and 25°C is approximately 20 to 80 mg/L. On the other hand, the liquid phase can contribute to the stability of the ethylenically unsaturated groups as long as dissolved oxygen gas (O₂) is substantially present therein. To prevent the decrease of the isocyanate group-containing, ethylenically unsaturated compounds by radical generation, the oxygen gas (O₂) concentration in the liquid phase in the invention is controlled to be in the range of 0.5 mg/L to 80 mg/L, preferably 2 mg/L to 70 mg/L, and more preferably 6 mg/L to 50 mg/L.

For long-term storage, it is preferable that oxygen gas (O₂) is dissolved in the liquid phase in advance and the system is tightly closed to ensure that the compound can be stored with the above oxygen concentration. In this case, the liquid phase preferably contains oxygen gas (O₂) dissolved therein in advance at 0.5 mg/L to 80 mg/L, preferably 5 mg/L to 70 mg/L, and more preferably 10 mg/L to 50 mg/L.

The controlling of the oxygen gas (O₂) concentration in the liquid phase preferably includes:
a step of measuring the oxygen gas (O₂) concentration in the liquid phase;
a step of controlling the supply amount of oxygen gas (O₂) by controlling the introduction of the gas containing oxygen gas (O₂), based on the result of the measurement; and
a step of introducing the gas containing oxygen gas (O₂) to the liquid phase.

### (Method of measuring oxygen gas (O₂) concentration in liquid phase organic compound)

The oxygen gas (O₂) concentration in the liquid phase may be measured by conventional methods for the determination of the oxygen gas (O₂) concentration in a liquid phase. Direct measurement of the liquid phase is preferable. Alternatively, a portion of the liquid phase may be sampled and analyzed while maintaining the gas-liquid equilibrium state.

Means for directly measuring the liquid phase, in particular for the measurement of the dissolved oxygen gas (O₂) concentration in an organic solvent having a low water content, may be a commercially available dissolved oxygen meter for oxygen dissolved in organic solvents. For example, polarographic electrode UC-12-SOL manufactured by Central Kagaku Corp. may be used. The electrode is soaked in the liquid phase, and the oxygen gas (O₂) concentration in the liquid phase is measured. For the calibration of the electrode, a liquid containing a target isocyanate group-containing, ethylenically unsaturated compound is sufficiently bubbled with dry air and is thereafter analyzed with the electrode, and the liquid is then analyzed by another established determination method, the calibration being made based on the data obtained. Examples of such established determination methods include gas chromatography (GC). An exemplary GC measurement is described below.

Chromatograph: GC-9A (manufactured by Shimadzu Corporation)
Columns: Porapak Q 50/80 mesh, 0.5 m in length and 3.0 mm in diameter, and Molecular Sieve 5A 30/60 mesh, 3 m in length and 3.0 mm in diameter. These columns are connected in series.
Carrier gas: helium
Detector type: TCD
Temperature: injection inlet 200°C, column constant at 40°C, detector 150°C
A calibration curve is obtained using dry air diluted with dry nitrogen.

### (Control of oxygen supply amount by controlling introduction of gas containing oxygen gas (O₂) based on results of measurement of oxygen gas (O₂) concentration in liquid phase)

After the oxygen gas (O₂) concentration in the liquid phase is measured as described above, the supply amount of oxygen gas (O₂) is controlled by controlling the introduction of the gas containing oxygen gas (O₂), based on the measurement results so that the oxygen gas (O₂) concentration in the liquid phase is within the aforementioned range.

The oxygen gas (O₂) concentration in the liquid phase is probably made up of a correlation of factors such as the flow rate of the gas containing oxygen gas (O₂), the oxygen concentration in the gas containing oxygen gas (O₂), and the dissolution efficiency of oxygen gas (O₂) into the liquid phase.

Accordingly, conventional methods of controlling the above factors may be used without limitation as means for controlling the supply amount of oxygen gas (O₂) through the introduction of the gas containing oxygen gas (O₂). In detail, the dissolution efficiency of oxygen gas may be improved by optimizing equipment, for example by optimizing the shape of a nozzle which blows oxygen into the liquid phase such that the dissolution rate will not be a rate-determining factor. Alternatively, the oxygen concentration in the gas containing oxygen gas (O₂) may be controlled by controlling the oxygen concentration in the oxygen gas (O₂)-containing gas that is blown into the liquid phase. Still alternatively, the amount of the oxygen gas (O₂)-containing gas that is supplied to the liquid phase may be controlled by controlling the gas flow rate. Alternatively, the dissolution efficiency of the oxygen gas into the liquid phase may be controlled by controlling the pressure. At least one of these automatic controlling methods in conventional production facilities may be used in the invention.

For example, the oxygen supply amount may be controlled by controlling the flow rate of the gas containing oxygen gas (O₂) in a manner such that the current concentration of oxygen gas (O₂) in the liquid phase is measured and compared with the target concentration, and when the current concentration is lower than the target concentration, the flow rate of the oxygen gas (O₂)-containing gas blown into the liquid phase is temporarily increased by, for example, PID control (a type of feedback control) to achieve an oxygen supply amount that gives the target oxygen gas (O₂) concentration.

The concentration of oxygen gas (O₂) dissolved in the liquid phase may be controlled to the desired range by the methods as described hereinabove.

### (Method of introducing oxygen gas (O₂)-containing gas into liquid phase)

The gas containing oxygen gas (O₂) may be introduced into the liquid phase by any methods without limitation. For example, the gas containing oxygen gas (O₂) may be introduced into the liquid phase through a gas inlet tube from a dry air pipe or cylinder in a factory.

The liquid phase temperature and the pressure in the system are not particularly limited. The oxygen gas (O₂) may be introduced at room temperature or under heating, and at atmospheric pressure or reduced pressure.

### (Steps in which oxygen gas (O₂) concentration in liquid phase is controlled)

The steps in which the oxygen gas (O₂) concentration in the liquid phase is controlled may be any steps where the isocyanate group-containing, ethylenically unsaturated carboxylate compound is present in the liquid phase. In particular, the steps requiring this control include steps in which the isocyanate group-containing, ethylenically unsaturated carboxylate compound has high possibility of polymerization (such as heating, reaction, distillation and storage of the products in tanks), and steps in which the concentration of oxygen gas (O₂) dissolved in the liquid phase is likely to be lowered (such as at reduced pressure or distillation). Accordingly, the liquid phase requiring this control is for example a liquid phase in a reaction tank in which an isocyanate group-containing, ethylenically unsaturated carboxylate compound is formed, or a bottom liquid in a distillation step in which an isocyanate group-containing, ethylenically unsaturated carboxylate compound is purified. It is generally difficult to provide containers containing the products (for example, drums and tank trucks) with equipment for controlling the dissolved oxygen gas (O₂) concentration. Accordingly, it is preferable for higher stability during storage that the dissolved oxygen gas (O₂) concentration is sufficiently increased before the products are placed in the containers.

### (Processes for producing isocyanate group-containing, ethylenically unsaturated carboxylate compounds)

The isocyanate group-containing, ethylenically unsaturated carboxylate compounds may be produced by conventional production processes while involving the polymerization inhibition process according to the present invention. The conventional production processes include the modified Reppe process and propylene direct oxidation process in accordance with Kaitei Seizou Koutei Zu Zenshuu (revised edition of a complete collection of production steps) II (published from Kagaku Kogyo Sha, edited by the Society of Process Flow Sheet, Ministry of International Trade and Industry). (Meth)acryloyl group-containing isocyanate compounds are preferred isocyanate group-containing, ethylenically unsaturated carboxylate compounds, and they may be produced according to processes disclosed in JP-A-2006-232797 and JP-A-2007-55993.

The aforementioned process of inhibiting polymerization of isocyanate group-containing, ethylenically unsaturated carboxylate compounds may be incorporated in the processes for producing isocyanate group-containing, ethylenically unsaturated carboxylate compounds.

A detailed description is given below.

(1) Isocyanate group-containing, (meth)acrylate derivatives represented by Formula (A) below (hereinafter, also the compounds (A) ) may be produced by a process including a step of dehydrochlorinating an isocyanate group-containing, 3-chloropropionate derivative represented by Formula (a1) below (hereinafter, also the compound (a1)) in the presence of a basic nitrogen compound while controlling the oxygen gas (O₂) concentration in a liquid phase to not less than 0.5 mg/L.

Cl-CH₂-CHR⁵-COO-R⁶-NCO (a1)

CH₂=CR⁵-COO-R⁶-NCO (A)

In Formulae (A) and (a1), R⁵ is a hydrogen atom or a methyl group, and R⁶ is a C1-10, optionally branched alkylene group, a hydrocarbon group wherein a C3-6 cycloalkylene group has C0-3 alkylene groups at front and back ends thereof, or a C6-8 aromatic hydrocarbon ring.

In the above formulae, R⁶ is preferably a C1-10, optionally branched alkylene group, more preferably -CH₂-CH₂-(ethylene group) or -CH₂-CH₂-CH₂- (propylene group), and particularly preferably -CH₂-CH₂- (ethylene group).

The compounds (a1) may be produced by, for example, a process described in JP-A-2006-232797.

The dehydrochlorination is usually performed in the presence of a basic compound. A compound containing basic nitrogen is generally used as the basic compound. If a hydrogen atom is present on the nitrogen atom, it can react with the isocyanate group of a raw material or a target compound, with the result that the yield can be lowered or the basicity of the nitrogen atom can be lost. Accordingly, a basic nitrogen compound having tertiary nitrogen is preferably used.

In the present invention, the reaction liquid from the synthesis of the compound (a1) may be used directly in the dehydrochlorination. Alternatively, the compound (a1) may be purified by a method such as distillation and then be dehydrochlorinated. In view of industrial aspects, the former embodiment has advantages such as fewer steps.

If the basic nitrogen compound remains in the product containing the compound (A) that is obtained by the dehydrochlorination, the compound can be a cause of inducing the polymerization of the compound (A). The product is therefore preferably separated and purified by distillation.

Theoretically, the dehydrochlorination may be carried out using 1 molar equivalent of the basic nitrogen compound relative to 1 mol of the compound (a1). The basic nitrogen compound may be used in excess, but the excess of the basic nitrogen compound may accelerate polymerization depending on conditions. On the other hand, if the basic nitrogen compound is used in an excessively small amount, the compound (a1) remains unreacted and the reaction may not be completed. In particular, such consequences tend to result when the reaction liquid contains large amounts of alkali-decomposable chlorine derived from hydrogen chloride that occurs in the isocyanation.

Accordingly, an optimum usage amount of the basic nitrogen compound that will give the maximum isolation yield of the target compound (A) varies depending on reaction conditions or the like. In particular, when the reaction liquid from the synthesis of the compound (a1) is subjected to the dehydrochlorination directly without separating and purifying the compound (a1), the determination of an optimum usage amount of the basic nitrogen compound is difficult because a larger number of factors are involved.

In such cases, it is preferable that the usage amount of the basic nitrogen compound is determined based on the result of the measurement of the amount of the alkali-decomposable chlorine in the reaction liquid. In detail, the amount of the alkali-decomposable chlorine is measured and the basic nitrogen compound is used in 0.5 to 10 molar equivalents, preferably 0.8 to 5.0 molar equivalents, and more preferably 0.9 to 2.0 molar equivalents based on 1 mol of the alkali-decomposable chlorine. The dehydrochlorination according to this embodiment provides an isocyanate group-containing, (meth)acrylate derivative in a high isolation yield. In the present specification, the alkali-decomposable chlorine refers to chlorine that can be quantitatively determined under analytical conditions described later.

When the reaction liquid from the synthesis of the compound (a1) is purified by distillation or the like before the dehydrochlorination, the basic nitrogen compound may be used in 0.5 to 10 molar equivalents, preferably 0.8 to 5.0 molar equivalents, and more preferably 0.9 to 2.0 molar equivalents based on 1 mol of the compound (a1).

In the production processes according to the present invention, the dehydrochlorination is performed in the presence of the basic nitrogen compound at a certain level of constant temperature. Because the compound (A) produced can undergo polymerization at a high temperature, the reaction temperature is desirably in the range of 40 to 120°C, preferably 40 to 100°C, and more preferably 45 to 90°C.

The reaction time varies depending on, for example, the reaction temperature and the basicity of the basic nitrogen compound. It is generally about 10 minutes to 40 hours, preferably 30 minutes to 30 hours, and more preferably 1 hour to 10 hours.

In the reaction, a solvent that does not react with the isocyanate group may be used, with examples including aprotic solvents including hydrocarbons such as toluene and xylene; acetates such as ethyl acetate, propyl acetate and butyl acetate; and chlorine solvents such as methylene chloride. The solvent preferably has a lower boiling point than the product.

In the above production step, the oxygen gas (O₂) concentration in the liquid phase is measured, and oxygen gas (O₂) is introduced into the liquid phase so that the oxygen gas (O₂) concentration in the liquid phase is not less than 0. 5 mg/L. Here, the oxygen gas (O₂) concentration in the liquid phase may be controlled to be not less than 0.5 mg/L by the method as described hereinabove.

After the dehydrochlorination, the hydrochloride is removed as required and the residual basic nitrogen compound is separated. Thereafter, the compound (A) is isolated by a method such as distillation, crystallization, extraction or column treatment, preferably by distillation. During the distillation, the oxygen gas (O₂) concentration in the liquid phase is controlled to not less than 0.5 mg/L in the similar manner as in the production step in order to prevent the polymerization of the product.

By the above isolation, isocyanate group-containing, (meth) acrylate derivatives can be obtained in a high yield with a hydrolyzable chlorine content of not more than 300 ppm.

In an embodiment, the isocyanate group-containing, (meth)acrylate derivative may be treated with the basic nitrogen compound as required, thereby reducing the hydrolyzable chlorine. In this treatment, the basic nitrogen compound may be used in 0.1 to 10 molar equivalents, preferably 0.2 to 5 molar equivalents, and more preferably 0.3 to 2 molar equivalents based on 1 mol of the hydrolyzable chlorine.

The treatment may be performed in the presence of the basic nitrogen compound at a certain level of constant temperature. Because the ester derivative can undergo polymerization at a high temperature, the treatment temperature is desirably in the range of 10 to 120°C, preferably 10 to 100°C, and more preferably 20 to 80°C. The oxygen gas (O₂) concentration in the liquid phase is controlled to not less than 0.5 mg/L in the similar manner as in the production step.

(2) Isocyanate group-containing, ethylenically unsaturated carboxylate compounds represented by Formula (B) (hereinafter, also the compounds (B)) may be produced by a process including a step of obtaining an isocyanate group-containing, ethylenically unsaturated carboxylate compound represented by Formula (B) by dehydrochlorinating an isocyanate compound represented by Formula (b1) (hereinafter, also the compound (b1)) in the presence of a basic nitrogen compound while controlling the oxygen gas (O₂) concentration in a liquid phase to not less than 0.5 mg/L.

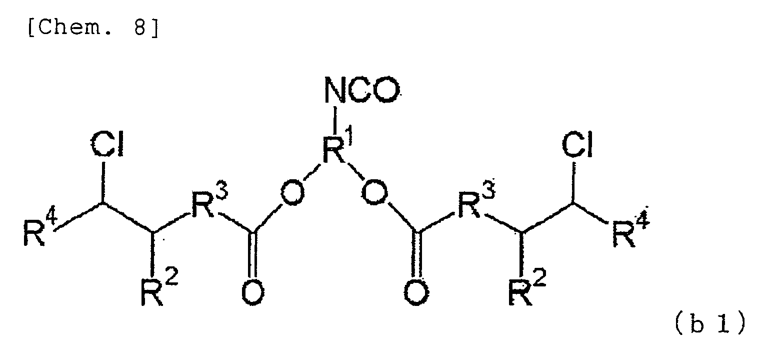

In the formula, R¹ is a C1-10, linear or branched saturated aliphatic group, each R² is independently a hydrogen atom or a methyl group, each R³ is independently a C0-5, linear or branched alkylene group, and each R⁴ is independently a hydrogen atom, a C1-6, linear or branched alkyl group, or an aryl group.

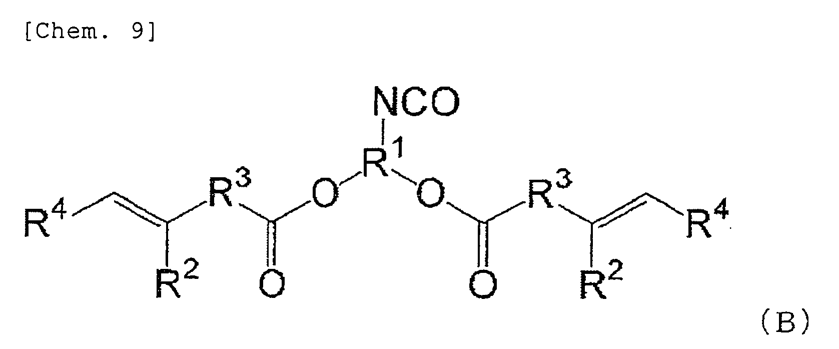

In the formula, R¹ to R⁴ are the same as described above. The compounds (b1) may be produced according to a process disclosed in JP-A-2007-55993.

The reaction temperature in the production step varies depending on the kinds of the compounds used, but is usually in the range of 0 to 150°C, and preferably 20 to 100°C. A low reaction temperature may reduce the reaction rate. If the reaction temperature is high, the unsaturated bonds formed by the dehydrochlorination may undergo polymerization.

Whether a solvent is used in the production step depends on the kinds of the compounds used. When the compound (b1) is liquid or can be molten, the reaction may be performed without using solvents. When the compound (b1) is solid or cannot be molten, the reaction preferably involves a solvent.

Specific examples of the solvents include esters such as methyl acetate, ethyl acetate, propyl acetate, isopropyl acetate and butyl acetate; linear ethers such as diethyl ether, dipropyl ether and dibutyl ether; cyclic ethers such as dioxane, dioxolane and tetrahydrofuran; aromatic hydrocarbons such as toluene, xylene, ethylbenzene, mesitylene and cumene; aliphatic hydrocarbons such as propane, hexane, heptane and cyclohexane; and halogenated hydrocarbons such as methylene chloride, 1,2-dichloroethane and 1,2-dichlorobenzene.

When the solvent is used, the usage amount thereof is usually such that the amount of the compound (b1) is 0.5 to 80% by mass, and preferably 5 to 50% by mass relative to the total mass of the compound (b1) and the solvent. If the amount of the solvent is insufficient, the reaction system cannot be stirred appropriately and the reaction rate may be lowered. Further, the removal of the salt formed may be difficult. An excessively large amount of the solvent does not affect the reaction but increases the amount of solvent waste, possibly increasing the burden on the environment.

In the production step, a common compound containing basic nitrogen may be used as the basic nitrogen compound. If a hydrogen atom is present on the basic nitrogen atom, it can react with the isocyanate group of the compound (b1), with the result that the yield can be lowered.

Therefore, the basic nitrogen compound is preferably a basic nitrogen compound having tertiary nitrogen. A weakly basic nitrogen compound such as quinoline in which an aromatic ring is bonded directly to the nitrogen atom is insufficient in basicity, and a certain level of basicity is required in order to carry out the dehydrochlorination efficiently. Accordingly, a preferred basic nitrogen compound contains tertiary nitrogen and the tertiary nitrogen atom has one or more substituent groups other than aromatic rings, for example alkyl groups. Further, it is preferable that the number of aromatic rings substituted on the tertiary nitrogen atom is 1 or less.

Specific examples of the basic nitrogen compounds used in the production step include trimethylamine, triethylamine, tripropylamine, dimethylethylamine, dimethylisopropylamine, diethylmethylamine, dimethylbutylamine, dimethylhexylamine, diisopropylethylamine, dimethylcyclohexylamine, tetramethyldiaminomethane, dimethylbenzylamine, tetramethylethylenediamine, tetramethyl-1,4-diaminobutane, tetramethyl-1,3-diaminobutane, tetramethyl-1,6-diaminohexane, pentamethyldiethylenetriamine, 1-methylpiperidine, 1-ethylpiperidine, N,N-methylpiperazine, N-methylmorpholine, 1,8-diazabicyclo[5.4.0]-7-undecene (DBU), 1,5-diazabicyclo[4.3.0]-5-nonene (DBN), 2,4-diazabicyclo[2.2.2]octane (DABCO), N,N-dimethylaniline, N,N-diethylaniline, and ion exchange resins having tertiary nitrogen.

Of these, trimethylamine, triethylamine, tripropylamine and tetramethylenediamine are preferable. The above basic nitrogen compounds may be used singly, or two or more kinds may be used in combination.

The usage amount of the basic nitrogen compound in the production step varies depending on the kinds of the compounds used. In a usual embodiment, the amount of the alkali-decomposable chlorine present in the reaction liquid from the reaction in the production step is measured and the basic nitrogen compound is used in a 0.5 to 10-fold molar amount, preferably a 0.8 to 5.0-fold molar amount, and more preferably a 0.9 to 2.0-fold molar amount based on the amount of the alkali-decomposable chlorine. If the usage amount of the basic nitrogen compound is small, the yield may be lowered. If the usage amount of the basic nitrogen compound is large, the stability of the obtainable compound (B) may be lowered. Further, industrial production costs will be increased.

Herein, the amount of the alkali-decomposable chlorine is determined by diluting the reaction liquid from the production step with a methanol/water mixture solvent, adding an aqueous sodium hydroxide solution thereto followed by heating, and potentiometrically titrating the solution with a silver nitrate solution.

In the production step, the oxygen gas (O₂) concentration in the liquid phase is measured, and oxygen gas (O₂) is introduced into the liquid phase so that the oxygen gas (O₂) concentration in the liquid phase is not less than 0.5 mg/L. Here, the oxygen gas (O₂) concentration in the liquid phase may be controlled to not less than 0.5 mg/L by the method as described hereinabove.

The compound (B) obtained in the production step may be purified by common technique such as filtration, extraction, recrystallization or distillation. During the distillation, the oxygen gas (O₂) concentration in the liquid phase is controlled to not less than 0.5 mg/L in the similar manner as in the production step in order to prevent the polymerization of the product.

(3) Isocyanate group-containing, ethylenically unsaturated carboxylate compounds represented by Formula (B') wherein R² in Formula (B) is a methyl group (hereinafter, also the compounds (B')) may be produced by a process including a step of obtaining an ester compound of Formula (b4) (hereinafter, also the compound (b4)) by reacting a dihydroxyamine mineral acid salt compound of Formula (b2) (hereinafter, also the compound (b2)) and a compound of Formula (b3) (hereinafter, also the compound (b3)), and a step of obtaining a compound (B') by reacting the compound (b4) and a compound of Formula (b5) (hereinafter, also the compound (b5)), wherein the oxygen gas (O₂) concentration in the liquid phase in these steps is controlled to not less than 0.5 mg/L.

### [First step]

The first step is a step of obtaining a compound (b4) from a compound (b2) and a compound (b3).

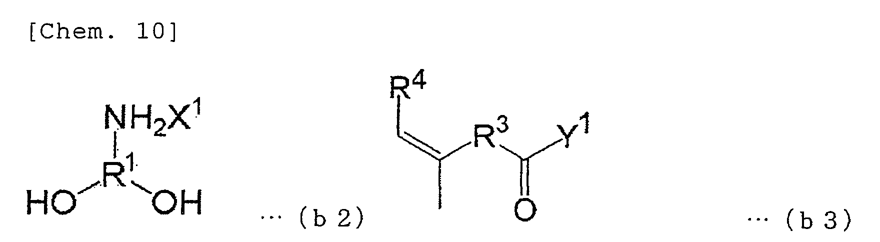

In the formulae, R¹ is a C1-10, linear or branched saturated aliphatic group, X¹ is a mineral acid, R³ is a C0-5, linear or branched alkylene group, R⁴ is a hydrogen atom, a C1-6, linear or branched alkyl group, or an aryl group, and Y¹ is a hydroxyl group, a chlorine atom or R⁶O- (wherein R⁶ is a C1-6 alkyl group).

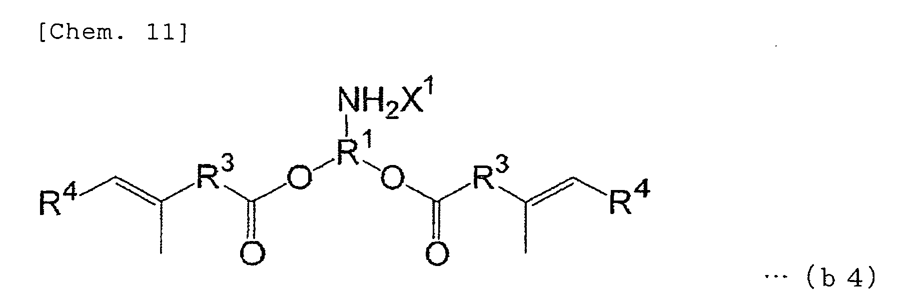

In the formula, R¹, R³, R⁴ and X¹ are as described above, and each R³ or R⁴ is an independent group.

### [Second step]

The second step is a step of obtaining a compound (B') from the compound (b4) and a compound (b5).

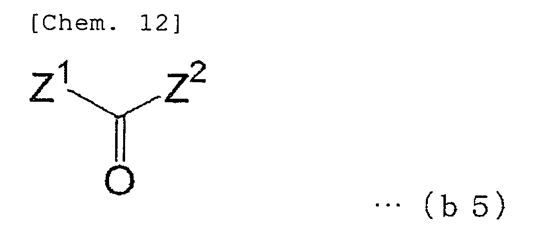

In the formula, Z¹ and Z² are each independently a chlorine atom, a bromine atom, R⁷O- (wherein R⁷ is a C1-6, linear or branched alkyl group, a C1-6, linear or branched alkenyl group or an optionally substituted aryl group), an imidazole residue or a pyrazole residue.

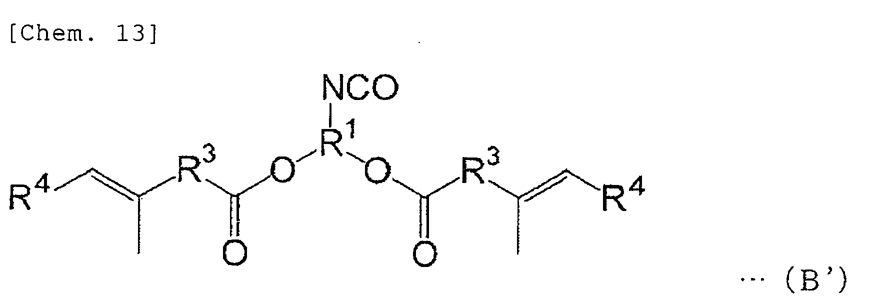

In the formula, R¹, R³ and R⁴ are the same as described above.
The compounds (b2) may be produced according to a process disclosed in JP-A-2007-55993.

### (3-i) First step

The compounds (b3) used in the first step may be readily available in the market. Specific examples of the compounds (b3) include methacrylic acid, 3-methyl-3-butenoic acid, tiglic acid, 4-methyl-4-pentenoic acid, α-methylcinnamic acid, acid chloride compounds of the above carboxylic acids, and ester compounds formed between the above carboxylic acids and C1-6, linear or branched alcohol compounds such as methyl esters, ethyl esters, propyl esters, isopropyl esters, butyl esters, isobutyl esters, pentyl esters, hexyl esters and cyclohexyl esters.

The compounds (b3) may be carboxylic acid chloride compounds of the above carboxylic acids. Methods for producing carboxylic acid chloride compounds from the carboxylic acids are known in the art. For example, JP-B-S57-026497, JP-A-S52-089617 and JP-A-H11-199540 disclose methods of obtaining a carboxylic acid chloride compound from a carboxylic acid and thionyl chloride, phosphorus pentachloride or phosgene.

The reaction temperature in the first step varies depending on the kinds of the compounds used, but is usually in the range of 30 to 150 °C, and preferably 50 to 120 °C. A low reaction temperature may reduce the reaction rate. If the reaction temperature is high, increased amounts of impurities may be caused and the unsaturated bonds may undergo polymerization.

Whether a solvent is used in the first step depends on, for example, the kinds of the compounds used. When the compound (b2) and/or the compound (b3) and/or the compound (b4) are liquid or can be molten, the reaction may be performed without using solvents. When the compound (b2) and/or the compound (b3) and/or the compound (b4) are solid or cannot be molten, the reaction preferably involves a solvent.

Specific examples of the solvents include esters such as methyl acetate, ethyl acetate, propyl acetate, isopropyl acetate and butyl acetate; linear ethers such as diethyl ether, dipropyl ether and dibutyl ether; cyclic ethers such as dioxane, dioxolane and tetrahydrofuran; aromatic hydrocarbons such as toluene, xylene, ethylbenzene, mesitylene and cumene; aliphatic hydrocarbons such as propane, hexane, heptane and cyclohexane; and halogenated hydrocarbons such as methylene chloride, 1,2-dichloroethane and 1,2-dichlorobenzene.

When the solvent is used, the usage amount thereof is usually such that the amount of the compound (b2) is 1 to 50% by mass, preferably 5 to 30% by mass, and more preferably 10 to 20% by mass relative to the total mass of the compound (b2), the compound (b3) and the solvent. If the amount of the solvent is insufficient, the reaction system cannot be stirred appropriately and the reaction rate may be lowered. An excessively large amount of the solvent does not affect the reaction but increases the amount of solvent waste, possibly increasing the burden on the environment.

The amount of the compound (b3) relative to the compound (b2) varies depending on the kinds of the compounds used, but is usually a 2 to 10-fold molar amount, and preferably a 2 to 5-fold molar amount. If the usage amount of the compound (b3) is small, the yield may be lowered and increased amounts of impurities may be caused. A large amount of the compound (b3) does not affect the reaction but increases wastes, possibly increasing the burden on the environment.

In the production step, the oxygen gas (O₂) concentration in the liquid phase is measured, and oxygen gas (O₂) is introduced into the liquid phase so that the oxygen gas (O₂) concentration in the liquid phase is not less than 0.5 mg/L. Here, the oxygen gas (O₂) concentration in the liquid phase may be controlled to not less than 0.5 mg/L by the method as described hereinabove.

The compound (b4) obtained in the first step may be purified by common technique such as extraction, recrystallization or distillation. During the distillation, the oxygen gas (O₂) concentration in the liquid phase is controlled to not less than 0.5 mg/L in the similar manner as in the production step in order to prevent the polymerization. The compound may be used in the reaction in the third step without purification.

### (3-ii) Second step

The reaction temperature in the second step varies depending on the kinds of the compounds used, but is usually in the range of 30 to 150°C, and preferably 50 to 120°C. A low reaction temperature may reduce the reaction rate. If the reaction temperature is high, increased amounts of impurities may be caused and the unsaturated bonds may undergo polymerization.

Whether a solvent is used in the second step depends on, for example, the kinds of the compounds used. When the compound (b4) is liquid or can be molten, the reaction may be performed without using solvents. When the compound (b4) is solid or cannot be molten, the reaction preferably involves a solvent.

Specific examples of the solvents include esters such as methyl acetate, ethyl acetate, propyl acetate, isopropyl acetate and butyl acetate; linear ethers such as diethyl ether, dipropyl ether and dibutyl ether; cyclic ethers such as dioxane, dioxolane and tetrahydrofuran; aromatic hydrocarbons such as toluene, xylene, ethylbenzene, mesitylene and cumene; aliphatic hydrocarbons such as propane, hexane, heptane and cyclohexane; and halogenated hydrocarbons such as methylene chloride, 1,2-dichloroethane and 1,2-dichlorobenzene.

When the solvent is used, the usage amount thereof is usually such that the amount of the compound (b4) is 0.5 to 80% by mass, and preferably 5 to 50% by mass relative to the total mass of the compound (b4), the compound (b5) and the solvent. If the amount of the solvent is insufficient, the reaction system cannot be stirred appropriately and the reaction rate may be lowered. An excessively large amount of the solvent does not affect the reaction but increases the amount of solvent waste, possibly increasing the burden on the environment.

Specific examples of Z¹ and Z² in the compounds (b5) used in the second step are described in JP-A-2007-55993. Dimers or trimers of the compounds (b5) may also be used.

The usage amount of the compound (b5) relative to the compound (b4) varies depending on the kinds of the compounds used. Theoretically, however, the reaction between the compound (b4) and the compound (b5) takes place in a 1:1 molar ratio. For smooth reaction, it is preferable that the compound (b5) is used in an excess amount. The usage amount of the compound (b5) relative to the compound (b4) is usually a 1 to 10-fold molar amount, and preferably a 1 to 5-fold molar amount. If the usage amount of the compound (b5) is small, unreacted compound (b4) remains and the yield may be lowered. Further, increased amounts of impurities may be caused. A large amount of the compound (b5) does not affect the reaction but may require special removal apparatus and may increase the burden on the environment.

In the production step, the oxygen gas (O₂) concentration in the liquid phase is measured, and oxygen gas (O₂) is introduced into the liquid phase so that the oxygen gas (O₂) concentration in the liquid phase is not less than 0.5 mg/L. Here, the oxygen gas (O₂) concentration in the liquid phase may be controlled to not less than 0.5 mg/L by the method as described hereinabove.

The compound (B') obtained in the second step may be purified by common technique such as extraction, recrystallization or distillation. During the distillation, the oxygen gas (O₂) concentration in the liquid phase is controlled to not less than 0.5 mg/L in the similar manner as in the production step in order to prevent the polymerization.

### EXAMPLES

The present invention will be described in detail by presenting examples hereinbelow without limiting the scope of the invention.

Measurement conditions in the examples are as follows.

### (Monomer analysis conditions: gas chromatography)

Column: DB-1 (30 m in length x 0.32 mm in inner diameter x 1 µm in thickness) manufactured by J&W
Sample injection part temperature: 300°C
Detector temperature: 300°C
Detector: FID (hydrogen flame ionization detector)
Temperature-increasing program: 50°C → temperature increase at 10°C/min → 320°C (held constant for 5 minutes)
Flow rate: 1.2 mL/min
Sample dilution solvent: methylene chloride

### [Reference Example 1]

Methyl acrylate monomer (a reagent manufactured by JUNSEI CHEMICAL CO., LTD.) weighing 100 g was placed in a 300 mL volume glass flask equipped with a gas inlet tube (a glass single tube 4 mm in inner diameter), a cooling condenser (which had a gas outlet open to atmospheric pressure and was equipped with a particulate calcium chloride tube), a thermometer and a calibrated dissolved oxygen meter for organic compounds (UC-12-SOL manufactured by Central Kagaku Corp.). The monomer was heated to 100°C in an oil bath. Dry air (oxygen gas (O₂) concentration: 21% by volume) was introduced at 1 mL/min through the gas inlet tube, and the residual amount of the methyl acrylate monomer was measured by gas chromatography over a period of 10 hours. The measurement showed that the monomer reduced at approximately 0.12%/h on average. This monomer decrease was attributed to polymerization (oligomerization) by heat. During the above procedures, the electrode constantly indicated a dissolved oxygen gas (O₂) concentration in the range of 30 to 32 mg/L.

### [Comparative Example 1]

The procedures in Reference Example 1 were repeated except that the dry air was replaced by dry air diluted twenty times by volume with dry nitrogen (oxygen gas (O₂) concentration: 1% by volume), and the residual amount of the methyl acrylate monomer was measured over a period of 10 hours. The measurement showed that the monomer reduced at approximately 0.75%/h on average. During the above procedures, the electrode of the oxygen gas (O₂) concentration meter constantly indicated a dissolved oxygen gas (O₂) concentration in the range of 0.2 to 0.3 mg/L.

### [Reference Example 2]

The procedures in Reference Example 1 were repeated except that the methyl acrylate monomer was replaced by a reaction solution of methyl acrylate (methyl acrylate content: 83% by mass), and the residual amount of the methyl acrylate monomer was measured over a period of 10 hours. The measurement showed that the monomer reduced at approximately 0.14%/h on average. During the above procedures, the electrode constantly indicated a dissolved oxygen gas (O₂) concentration in the range of 28 to 29 mg/L.

### [Reference Example 3]

The procedures in Reference Example 1 were repeated except that the gas outlet at the tip of the cooling condenser was not open to atmospheric pressure but was connected to a vacuum pump, and a reduced pressure reactor was created by controlling the pressure with nitrogen through a needle valve such that a mercury manometer indicated 100 mm Hg. The residual amount of the methyl acrylate monomer was measured over a period of 10 hours. The measurement showed that the monomer reduced at approximately 0.17%/h on average. During the above procedures, the electrode constantly indicated a dissolved oxygen gas (O₂) concentration in the range of 4 to 5 mg/L.

### [Comparative Example 2]

The procedures in Reference Example 3 were repeated except that the dry air was replaced by dry air diluted twenty times by volume with dry nitrogen (oxygen gas (O₂) concentration: 1% by volume), and the residual amount of the methyl acrylate monomer was measured over a period of 10 hours. The measurement showed that the monomer reduced at approximately 0.89%/h on average. During the above procedures, the electrode constantly indicated a dissolved oxygen gas (O₂) concentration in the range of 0.1 to 0.2 mg/L.

### [Reference Example 4]

The procedures in Reference Example 3 were repeated except that the dry air at 1 mL/min was replaced by dry air diluted five times by volume with dry nitrogen (oxygen gas (O₂) concentration: 4% by volume) at 5 mL/min, and the residual amount of the methyl acrylate monomer was measured over a period of 10 hours. The measurement showed that the monomer reduced at approximately 0.21%/h on average. During the above procedures, the electrode of the oxygen concentration meter constantly indicated a dissolved oxygen gas (O₂) concentration in the range of 1.1 to 1.3 mg/L.

### [Example 1]

The procedures in Reference Example 3 were repeated except that the methyl acrylate monomer was replaced by 2-acryloyloxyethyl isocyanate (KARENZ AOI (registered trademark) manufactured by SHOWA DENKO K.K., purity by gas chromatography: 99.7%), and the residual amount of the 2-acryloyloxyethyl isocyanate monomer was measured over a period of 10 hours. The measurement showed that the monomer reduced at approximately 0.08%/h on average. During the above procedures, the electrode of the oxygen concentration meter constantly indicated a dissolved oxygen gas (O₂) concentration in the range of 6.5 to 7.0 mg/L.

### [Comparative Example 3]

The procedures in Example 1 were repeated except that the dry air was replaced by dry air diluted twenty times by volume with dry nitrogen (oxygen gas (O₂) concentration: 1% by volume), and the residual amount of the 2-acryloyloxyethyl isocyanate monomer was measured over a period of 10 hours. The measurement showed that the monomer reduced at approximately 1.1%/h on average. During the above procedures, the electrode of the oxygen concentration meter constantly indicated a dissolved oxygen gas (O₂) concentration in the range of 0.1 to 0.3 mg/L.

### [Example 2]

The procedures in Example 1 were repeated except that the isocyanate group-containing, ethylenically unsaturated compound monomer, i.e., 2-acryloyloxyethyl isocyanate (KARENZ AOI (registered trademark) manufactured by SHOWA DENKO K.K., purity by gas chromatography: 99.7%) was replaced by a reaction liquid of 2-acryloyloxyethyl isocyanate (purity by gas chromatography: 78.8%), and the residual amount of the 2-acryloyloxyethyl isocyanate monomer was measured over a period of 10 hours. The measurement showed that the monomer reduced at approximately 0.16%/h on average. During the above procedures, the electrode of the oxygen concentration meter constantly indicated a dissolved oxygen gas (O₂) concentration in the range of 4.8 to 5.2 mg/L.

### [Comparative Example 4]

The procedures in Example 2 were repeated except that the dry air was replaced by dry air diluted twenty times by volume with dry nitrogen (oxygen gas (O₂) concentration: 1% by volume), and the residual amount of the 2-acryloyloxyethyl isocyanate monomer was measured over a period of 10 hours. The measurement showed that the monomer reduced at approximately 0.97%/h on average. During the above procedures, the electrode of the oxygen concentration meter constantly indicated a dissolved oxygen gas (O₂) concentration in the range of 0.2 to 0.3 mg/L.

### [Example 3]

100 g of a reaction solution of 2-acryloyloxyethyl isocyanate (AOI) (purity by gas chromatography: 78.8%) was placed in a 300 mL volume glass flask equipped with a gas inlet tube (a glass single tube 4 mm in inner diameter), an Allihn cooling condenser (which had a gas outlet connected to a vacuum pump, a pressure control valve and a pressure gauge), a thermometer and a calibrated dissolved oxygen meter for organic compounds (UC-12-SOL manufactured by Central Kagaku Corp.). The solution was heated in an oil bath at 100°C. While dry air (oxygen gas (O₂) concentration: 21% by volume) was introduced at 1 mL/min through the gas inlet tube, the inside of the reaction vessel was brought to 2.2 kPa by means of the vacuum pump and the pressure control valve. The inner liquid temperature at this time was 92°C, and AOI was refluxed with the cooling tube like a bottom liquid in a distillation column. The residual amount of the AOI monomer was measured by gas chromatography over a period of 10 hours. The measurement showed that the monomer reduced at approximately 0.58%/h on average. During the above procedures, the electrode of the oxygen concentration meter constantly indicated a dissolved oxygen gas (O₂) concentration in the range of 1.8 to 2.0 mg/L.

### [Comparative Example 5]

The procedures in Example 3 were repeated except that the dry air was replaced by dry air diluted ten times by volume with dry nitrogen (oxygen gas (O₂) concentration: 2% by volume), and the residual amount of the AOI monomer was measured over time. The inner liquid was solidified and polymerization occurred in 8 hours. The reduction rate of the AOI monomer at the lapse of 5 hours was approximately 0.97%/h. During the above procedures, the electrode of the oxygen concentration meter constantly indicated a dissolved oxygen gas (O₂) concentration in the range of 0.1 to 0.2 mg/L.

### [Example 4]

The procedures in Example 2 were repeated except that the dry air at 1 mL/min was replaced by dry air diluted five times by volume with dry nitrogen (oxygen gas (O₂) concentration: 4% by volume) at 5 mL/min, and the residual amount of the 2-acryloyloxyethyl isocyanate monomer was measured over time. The measurement showed that the monomer reduced at approximately 0.35%/h. During the above procedures, the electrode of the oxygen concentration meter constantly indicated a dissolved oxygen gas (O₂) concentration in the range of 0.9 to 1.2 mg/L.

### [Example 5]

The procedures in Example 2 were repeated except that the dry air was replaced by diluted dry oxygen having an oxygen gas (O₂) concentration of 40% by volume (dilution gas: dry nitrogen), and the residual amount of the 2-acryloyloxyethyl isocyanate monomer was measured over time. The measurement showed that the monomer reduced at approximately 0.02%/h. During the above procedures, the electrode of the oxygen concentration meter constantly indicated a dissolved oxygen gas (O₂) concentration in the range of 10 to 11 mg/L.

### [Reference Example 5]

Dissolved oxygen gas (O₂) in methyl acrylate obtained by vacuum distillation (purity by gas chromatography: 99.8%) was quantitatively determined with a GC-calibrated dissolved oxygen meter for organic solvents (UC-12-SOL manufactured by Central Kagaku Corp.) at room temperature in atmospheric atmosphere, resulting in 0.3 mg/L. 0.8 kg of the methyl acrylate was placed in a SUS container (a 1-L can), and dry air (oxygen gas (O₂) concentration: 21% by volume) was blown thereto at 1 L/min through a glass tube. The dissolved oxygen gas (O₂) concentration was measured with the dissolved oxygen meter every 30 minutes, and the dissolved oxygen gas (O₂) concentration stabilized at 38 mg/L after 1.5 hours. The 1-L can was tightly closed with a cap and was stored at a constant temperature of 30°C. The residual amount of the methyl acrylate after the storage for one year was determined by gas chromatography, which showed that 99.4% remained after the 1-year storage. This monomer decrease was attributed to polymerization (oligomerization) during the storage. The dissolved oxygen gas (O₂) concentration according to the dissolved oxygen meter was 18 mg/L.

### [Comparative Example 6]

The methyl acrylate was stored in the same manner as in Reference Example 5 except that the 1-L can was tightly closed with a cap without blowing dry air. The methyl acrylate polymerized in the container on Day 192 after the initiation of the storage, and the content spouted from the container mouth due to the polymerization heat. The content consisted of a liquid and a popcorn-like solid (a polymer).

### [Example 6]

Dissolved oxygen gas (O₂) in 2-acryloyloxyethyl isocyanate obtained by vacuum distillation (purity by gas chromatography: 99.6%) was quantitatively determined as described in Reference Example 5, resulting in 0.3 mg/L. 1.0 kg of the 2-acryloyloxyethyl isocyanate was placed in a SUS container (a 1-L can), and dry air (oxygen gas (O₂) concentration: 21% by volume) was blown thereto at 1 L/min through a glass tube. The dissolved oxygen gas (O₂) concentration was measured with the dissolved oxygen meter every 30 minutes, and the dissolved oxygen gas (O₂) concentration stabilized at 41 mg/L after 1 hour. The 1-L can was tightly closed with a cap and was stored at a constant temperature of 30°C. The residual amount of the 2-acryloyloxyethyl isocyanate after the storage for one year was determined by gas chromatography, which showed that 98.4% remained after the 1-year storage. The dissolved oxygen gas (O₂) concentration according to the dissolved oxygen meter was 21 mg/L.

### [Comparative Example 7]

The 2-acryloyloxyethyl isocyanate was stored in the same manner as in Example 6 except that the 1-L can was tightly closed with a cap without blowing dry air. The residual amount after the storage for one year was 82.8%, and the dissolved oxygen gas (O₂) concentration according to the dissolved oxygen meter was 0.1 mg/L.

### [Example 7]

The procedures in Example 6 were repeated except that the dry air (oxygen gas (O₂) concentration: 21% by volume) was replaced by dry air diluted two times with dry nitrogen (oxygen gas (O₂) concentration: 10% by volume). The dissolved oxygen gas (O₂) concentration was measured with the dissolved oxygen meter every 30 minutes from the initiation of the blowing of the diluted air. The dissolved oxygen gas (O₂) concentration stabilized at 20 mg/L after 2.5 hours. The 1-L can was tightly closed with a cap and was stored at a constant temperature of 30°C. The residual amount of the 2-acryloyloxyethyl isocyanate after the storage for one year was determined by gas chromatography, which showed that 93.2% remained after the 1-year storage. The dissolved oxygen gas (O₂) concentration according to the dissolved oxygen meter was 7 mg/L.

### [Example 8]

250 ml of toluene, 59 g (0.33 mol) of (2-isocyanatoethyl) 3-chloropropionate obtained by a method disclosed in JP-A-2006-232797 and 50 g (0.49 mol) of triethylamine (boiling point: 89.4°C) were placed in a three-necked flask equipped with a gas inlet tube (a glass single tube 4 mm in inner diameter), a cooling condenser (which had a gas outlet open to atmospheric pressure and was equipped with a particulate calcium chloride tube), a thermometer and a calibrated dissolved oxygen meter for organic compounds (UC-12-SOL manufactured by Central Kagaku Corp.). They were heated at 50°C with stirring for 6 hours, cooled to room temperature, and filtered to remove the hydrochloride formed. The yield of acryloyloxyethyl isocyanate in the filtrate was 86%. While dry air (oxygen gas (O₂) concentration: 21% by volume) was introduced to the reaction liquid at 1 mL/min through the gas inlet tube, the system was held at 50°C for 10 hours and the residual amount of the acryloyloxyethyl isocyanate was measured during the hours by gas chromatography. The measurement showed that the monomer reduced at approximately 0.07%/h on average. This monomer decrease was attributed to polymerization (oligomerization) by heat. During the above procedures, the electrode constantly indicated a dissolved oxygen gas (O₂) concentration in the range of 40 to 42 mg/L.

### [Comparative Example 8]

The procedures in Example 8 were repeated except that the dry air was replaced by dry air diluted twenty times by volume with dry nitrogen (oxygen gas (O₂) concentration: 1% by volume), and the residual amount of the acryloyloxyethyl isocyanate in the filtrate was measured over time. The measurement showed that the monomer reduced at approximately 0.44%/h on average. During the above procedures, the electrode of the oxygen concentration meter constantly indicated a dissolved oxygen gas (O₂) concentration in the range of 0.2 to 0.3 mg/L.

### [Example 9]

250 ml of toluene, 59 g (0.33 mol) of (2-isocyanatoethyl) 3-chloropropionate obtained by a method disclosed in JP-A-2006-232797 and 50 g (0.49 mol) of triethylamine (boiling point: 89.4°C) were placed in a three-necked flask equipped with a gas inlet tube (a glass single tube 4 mm in inner diameter), a cooling condenser (which had a gas outlet open to atmospheric pressure and was equipped with a particulate calcium chloride tube), a thermometer and a calibrated dissolved oxygen meter for organic compounds (UC-12-SOL manufactured by Central Kagaku Corp.). They were heated at 50°C with stirring for 6 hours, cooled to room temperature, and filtered to remove the triethylamine hydrochloride formed. Excess triethylamine and toluene were distilled away, and the residue was distilled (62 to 67°C/0.7 kPa) for 4 hours to give 39 g (0.28 mol) of acryloyloxyethyl isocyanate (boiling point: 200°C) (yield: 85%). During the reaction and the distillation, dry air (oxygen gas (O₂) concentration: 21% by volume) was introduced at 1 mL/min through the gas inlet tube and the electrode constantly indicated a dissolved oxygen gas (O₂) concentration in the range of 42 to 46 mg/L.

### [Comparative Example 9]

The reaction and the distillation in Example 9 were repeated except that the dry air was replaced by dry air diluted twenty times by volume with dry nitrogen (oxygen gas (O₂) concentration: 1% by volume), thereby obtaining 36 g (0.26 mol) of 2-acryloyloxyethyl isocyanate (yield: 79%). During the procedures, the electrode of the oxygen concentration meter constantly indicated a dissolved oxygen gas (O₂) concentration in the range of 0.2 to 3 mg/L.

### [Example 10]

The procedures in Example 1 were repeated except that the 2-acryloyloxyethyl isocyanate was replaced by 2-methacryloyloxyethyl isocyanate (KARENZ MOI (registered trademark) manufactured by SHOWA DENKO K.K., purity by gas chromatography: 99.8%), and the residual amount of the MOI monomer was measured over a period of 10 hours. The measurement showed that the monomer reduced at approximately 0.07%/h on average. During the above procedures, the electrode of the oxygen concentration meter constantly indicated a dissolved oxygen gas (O₂) concentration in the range of 6.6 to 7.0 mg/L.

### [Comparative Example 10]

The procedures in Example 10 were repeated except that the dry air was replaced by dry air diluted twenty times by volume with dry nitrogen (oxygen gas (O₂) concentration: 1% by volume), and the residual amount of the MOI monomer was measured over a period of 10 hours. The measurement showed that the monomer reduced at approximately 1.1%/h on average. During the above procedures, the electrode of the oxygen concentration meter constantly indicated a dissolved oxygen gas (O₂) concentration in the range of 0.1 to 0.4 mg/L.

### [Example 11]

The procedures in Example 3 were repeated except that the reaction liquid of 2-acryloyloxyethyl isocyanate was replaced by 2-methacryloyloxyethyl isocyanate (KARENZ MOI (registered trademark) manufactured by SHOWA DENKO K.K., purity by gas chromatography: 99.8%), and the residual amount of the MOI monomer was measured by gas chromatography over a period of 10 hours. The measurement showed that the monomer reduced at approximately 0.49% /h on average. During the above procedures, the electrode of the oxygen concentration meter constantly indicated a dissolved oxygen gas (O₂) concentration in the range of 2.0 to 2.2 mg/L.

### [Comparative Example 11]

The procedures in Example 11 were repeated except that the dry air was replaced by dry air diluted ten times by volume with dry nitrogen (oxygen gas (O₂) concentration: 2% by volume), and the residual amount of the MOI monomer was measured over a period of 10 hours. The measurement showed that the monomer reduced at approximately 2.1%/h on average. During the above procedures, the electrode of the oxygen concentration meter constantly indicated a dissolved oxygen gas (O₂) concentration in the range of 0.1 to 0.3 mg/L.

### [Example 12]

The procedures in Example 10 were repeated except that the dry air at 1 mL/min was replaced by dry air diluted five times by volume with dry nitrogen (oxygen gas (O₂) concentration: 4% by volume) at 5 mL/min, and the residual amount of the MOI monomer was measured over a period of 10 hours. The measurement showed that the monomer reduced at approximately 0.28%/h on average. During the above procedures, the electrode of the oxygen concentration meter constantly indicated a dissolved oxygen gas (O₂) concentration in the range of 1.1 to 1.3 mg/L.

### [Example 13]

The procedures in Example 10 were repeated except that the dry air was replaced by diluted dry oxygen having an oxygen gas (O₂) concentration of 40% by volume (dilution gas: dry nitrogen), and the residual amount of the MOI monomer was measured over a period of 10 hours. The measurement showed that the monomer reduced at approximately 0.02%/h on average. During the above procedures, the electrode of the oxygen concentration meter constantly indicated a dissolved oxygen gas (O₂) concentration in the range of 10 to 11 mg/L.

### [Example 14]

2-Methacryloyloxyethyl isocyanate (KARENZ MOI (registered trademark) manufactured by SHOWA DENKO K.K., purity by gas chromatography: 99.8%) was analyzed in the same manner as the 2-acryloyloxyethyl isocyanate was analyzed in Example 6, and the dissolved oxygen gas (O₂) concentration in the monomer solution was determined to be 0.3 mg/L. 1.0 kg of the MOI solution was placed in a SUS container (a 1-L can), and dry air (oxygen gas (O₂) concentration: 21% by volume) was blown thereto at 1 L/min through a glass tube. The dissolved oxygen gas (O₂) concentration was measured with the dissolved oxygen meter every 30 minutes, and the dissolved oxygen gas (O₂) concentration stabilized at 44 mg/L after 1 hour. The 1-L can was tightly closed with a cap and was stored at a constant temperature of 30°C. The residual amount of the MOI monomer after the storage for one year was determined by gas chromatography, which showed that 99.0% remained after the 1-year storage. The dissolved oxygen gas (O₂) concentration according to the dissolved oxygen meter was 17 mg/L.

### [Comparative Example 12]

The MOI solution was stored in the same manner as in Example 14 except that the 1-L can was tightly closed with a cap without blowing dry air. The residual amount after the storage for one year was 83.9%, and the dissolved oxygen gas (O₂) concentration according to the dissolved oxygen meter was 0.2 mg/L.

### [Example 15]

The procedures in Example 1 were repeated except that the 2-acryloyloxyethyl isocyanate was replaced by 1,1-bis(acryloyloxymethyl)ethyl isocyanate (KARENZ BEI (registered trademark) manufactured by SHOWA DENKO K.K., purity by gas chromatography: 99.2%), and the residual amount of the BEI monomer was measured over a period of 10 hours. The measurement showed that the monomer reduced at approximately 0.08%/h on average. During the above procedures, the electrode of the oxygen concentration meter constantly indicated a dissolved oxygen gas (O₂) concentration in the range of 6.5 to 6.7 mg/L.

### [Comparative Example 13]

The procedures in Example 15 were repeated except that the dry air was replaced by dry air diluted twenty times by volume with dry nitrogen (oxygen gas (O₂) concentration: 1% by volume), and the residual amount of the BEI monomer was measured over a period of 10 hours. The measurement showed that the monomer reduced at approximately 1.3%/h on average. During the above procedures, the electrode of the oxygen concentration meter constantly indicated a dissolved oxygen gas (O₂) concentration in the range of 0.1 to 0.2 mg/L.

### [Example 16]

The procedures in Example 3 were repeated except that the reaction liquid of 2-acryloyloxyethyl isocyanate was replaced by 1,1-bis(acryloyloxymethyl)ethyl isocyanate (KARENZ BEI (registered trademark) manufactured by SHOWA DENKO K.K., purity by gas chromatography: 99.2%), and the residual amount of the BEI monomer was measured by gas chromatography over a period of 10 hours. The measurement showed that the monomer reduced at approximately 0. 60%/h on average. During the above procedures, the electrode of the oxygen concentration meter constantly indicated a dissolved oxygen gas (O₂) concentration in the range of 1.7 to 1.9 mg/L.

### [Comparative Example 14]

The procedures in Example 16 were repeated except that the dry air was replaced by dry air diluted ten times by volume with dry nitrogen (oxygen gas (O₂) concentration: 2% by volume), and the residual amount of the BEI monomer was measured over a period of 10 hours. The measurement showed that the monomer reduced at approximately 2.3%/h on average. During the above procedures, the electrode of the oxygen concentration meter constantly indicated a dissolved oxygen gas (O₂) concentration in the range of 0.1 to 0.3 mg/L.

### [Example 17]

The procedures in Example 15 were repeated except that the dry air at 1 mL/min was replaced by dry air diluted five times by volume with dry nitrogen (oxygen gas (O₂) concentration: 4% by volume) at 5 mL/min, and the residual amount of the BEI monomer was measured over a period of 10 hours. The measurement showed that the monomer reduced at approximately 0.29%/h on average. During the above procedures, the electrode of the oxygen concentration meter constantly indicated a dissolved oxygen gas (O₂) concentration in the range of 1.3 to 1.5 mg/L.

### [Example 18]

The procedures in Example 15 were repeated except that the dry air was replaced by diluted dry oxygen having an oxygen gas (O₂) concentration of 40% by volume (dilution gas: dry nitrogen), and the residual amount of the BEI monomer was measured over a period of 10 hours. The measurement showed that the monomer reduced at approximately 0.04%/h on average. During the above procedures, the electrode of the oxygen concentration meter constantly indicated a dissolved oxygen gas (O₂) concentration in the range of 9 to 10 mg/L.

### [Example 19]

1,1-Bis(acryloyloxymethyl)ethyl isocyanate (KARENZ BEI (registered trademark) manufactured by SHOWA DENKO K.K., purity by gas chromatography: 99.2%) was analyzed in the same manner as the 2-acryloyloxyethyl isocyanate was analyzed in Example 6, and the dissolved oxygen gas (O₂) concentration in the monomer solution was determined to be 0.4 mg/L. 1.0 kg of the BEI solution was placed in a SUS container (a 1-L can), and dry air (oxygen gas (O₂) concentration: 21% by volume) was blown thereto at 1 L/min through a glass tube. The dissolved oxygen gas (O₂) concentration was measured with the dissolved oxygen meter every 30 minutes, and the dissolved oxygen gas (O₂) concentration stabilized at 39 mg/L after 1 hour. The 1-L can was tightly closed with a cap and was stored at a constant temperature of 30°C. The residual amount of the BEI monomer after the storage for one year was determined by gas chromatography, which showed that 98.3% remained after the 1-year storage. The dissolved oxygen gas (O₂) concentration according to the dissolved oxygen meter was 15 mg/L.

### [Comparative Example 15]

The BEI solution was stored in the same manner as in Example 19 except that the 1-L can was tightly closed with a cap without blowing dry air. The residual amount after the storage for one year was 80.4%, and the dissolved oxygen gas (O₂) concentration according to the dissolved oxygen meter was 0.1 mg/L.

## Claims

1. A process of inhibiting polymerization of isocyanate group-containing, ethylenically unsaturated carboxylate compounds, which comprises controlling the oxygen gas (O₂) concentration in a liquid phase containing an isocyanate group-containing, ethylenically unsaturated carboxylate compound to not less than 0.5 mg/L.

2. The process of inhibiting polymerization of isocyanate group-containing, ethylenically unsaturated carboxylate compounds according to claim 1, which comprises introducing a gas containing oxygen gas (O₂) to the liquid phase.

3. The process of inhibiting polymerization of isocyanate group-containing, ethylenically unsaturated carboxylate compounds according to claim 2, wherein the gas containing oxygen gas (O₂) is air, diluted air or diluted oxygen.

4. The process of inhibiting polymerization of isocyanate group-containing, ethylenically unsaturated carboxylate compounds according to claim 2 or 3, wherein the process comprises:
a step of measuring the oxygen gas (O₂) concentration in the liquid phase; and
a step of controlling the supply amount of oxygen gas (O₂) by controlling the introduction of the gas containing oxygen gas (O₂), based on the result of the measurement.

5. The process of inhibiting polymerization of isocyanate group-containing, ethylenically unsaturated carboxylate compounds according to any one of claims 1 to 4, wherein the oxygen gas (O₂) concentration in the liquid phase is measured with an electrode that is immersed in the liquid phase.

6. The process of inhibiting polymerization of isocyanate group-containing, ethylenically unsaturated carboxylate compounds according to any one of claims 1 to 5, wherein the liquid phase is a bottom liquid in a distillation step.

7. The process of inhibiting polymerization of isocyanate group-containing, ethylenically unsaturated carboxylate compounds according to any one of claims 1 to 6, wherein the isocyanate group-containing, ethylenically unsaturated carboxylate compound is a compound represented by Formula (A) or (B) below:
CH₂=CR⁵-COO-R⁶-NCO (A)
wherein R⁵ is a hydrogen atom or a methyl group, and R⁶ is a C1-10, optionally branched alkylene group, a hydrocarbon group wherein a C3-6 cycloalkylene group has C0-3 alkylene groups at front and back ends thereof, or a C6-8 aromatic hydrocarbon ring; 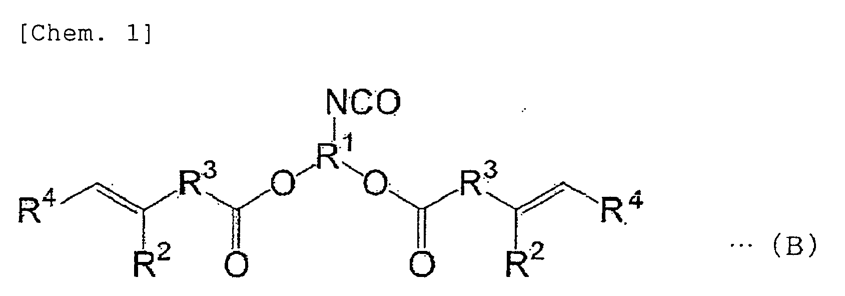 wherein R¹ is a C1-10, linear or branched saturated aliphatic group, each R² is independently a hydrogen atom or a methyl group, each R³ is independently a C0-5, linear or branched alkylene group, and each R⁴ is independently a hydrogen atom, a C1-6, linear or branched alkyl group, or an aryl group.

8. The process of inhibiting polymerization of isocyanate group-containing, ethylenically unsaturated carboxylate compounds according to claim 7, wherein the isocyanate group-containing, ethylenically unsaturated carboxylate compound is 2-methacryloyloxyethyl isocyanate, 2-acryloyloxyethyl isocyanate, 1,1-bis(acryloyloxymethyl)ethyl isocyanate, 2-(isocyanatoethyloxy)ethyl methacrylate or 2-(isocyanatoethyloxy)ethyl acrylate.

9. The process of inhibiting polymerization of isocyanate group-containing, ethylenically unsaturated carboxylate compounds according to any one of claims 1 to 8, wherein the water content in the liquid phase is not more than 5% by mass.

10. A process for producing isocyanate group-containing, ethylenically unsaturated carboxylate compounds, comprising performing the process of inhibiting polymerization described in any one of claims 1 to 9.

11. A process for producing isocyanate group-containing, ethylenically unsaturated carboxylate compounds represented by Formula (A), the process comprising:
a step of dehydrochlorinating an isocyanate group-containing, 3-chloropropionate derivative represented by Formula (a1) in the presence of a basic nitrogen compound; and
a step of controlling the oxygen gas (O₂) concentration in a liquid phase to not less than 0.5 mg/L;
Cl-CH₂-CHR⁵-COO-R⁶-NCO (a1)
CH₂=CR⁵-COO-R⁶-NCO (A)
wherein R⁵ is a hydrogen atom or a methyl group, and R⁶ is a C1-10, optionally branched alkylene group, a hydrocarbon group wherein a C3-6 cycloalkylene group has C0-3 alkylene groups at front and back ends thereof, or a C6-8 aromatic hydrocarbon ring.

12. A process for producing isocyanate group-containing, ethylenically unsaturated carboxylate compounds represented by Formula (B), the process comprising:
a step of dehydrochlorinating an isocyanate compound represented by Formula (b1) in the presence of a basic nitrogen compound; and
a step of controlling the oxygen gas (O₂) concentration in a liquid phase to not less than 0.5 mg/L; 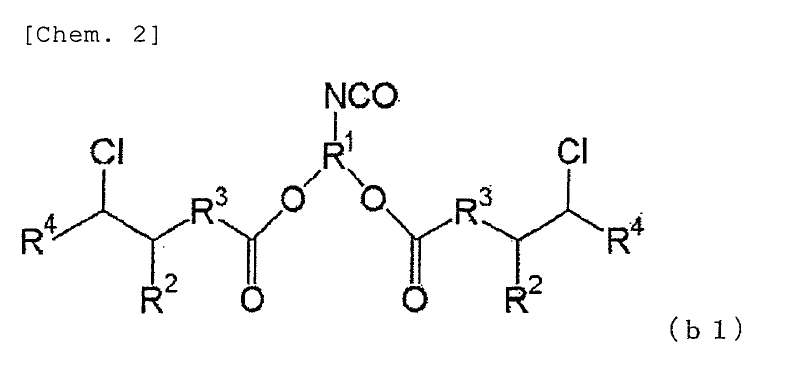
wherein R¹ is a C1-10, linear or branched saturated aliphatic group, each R² is independently a hydrogen atom or a methyl group, each R³ is independently a C0-5, linear or branched alkylene group, and each R⁴ is independently a hydrogen atom, a C1-6, linear or branched alkyl group, or an aryl group; 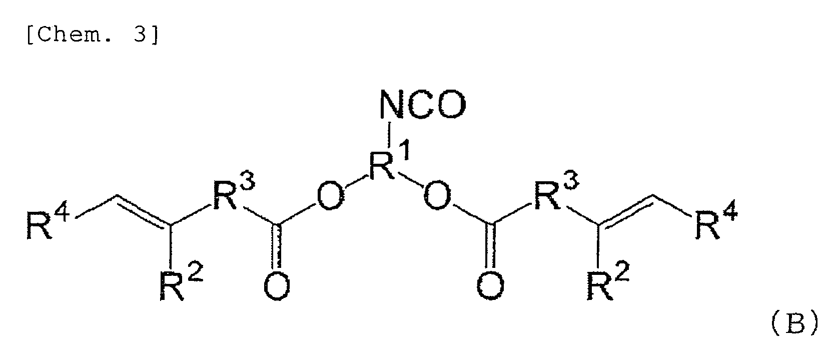
wherein R¹ to R⁴ are the same as above.

13. A process for producing isocyanate group-containing, ethylenically unsaturated carboxylate compounds, the process comprising:
a step of obtaining an ester compound of Formula (b4) from a dihydroxyamine mineral acid salt compound of Formula (b2) and a compound of Formula (b3);
a step of obtaining an isocyanate group-containing, ethylenically unsaturated carboxylate compound of Formula (B') from the ester compound of Formula (b4) and a compound of Formula (b5); and
a step of controlling the oxygen gas (O₂) concentration in a liquid phase to not less than 0.5 mg/L; 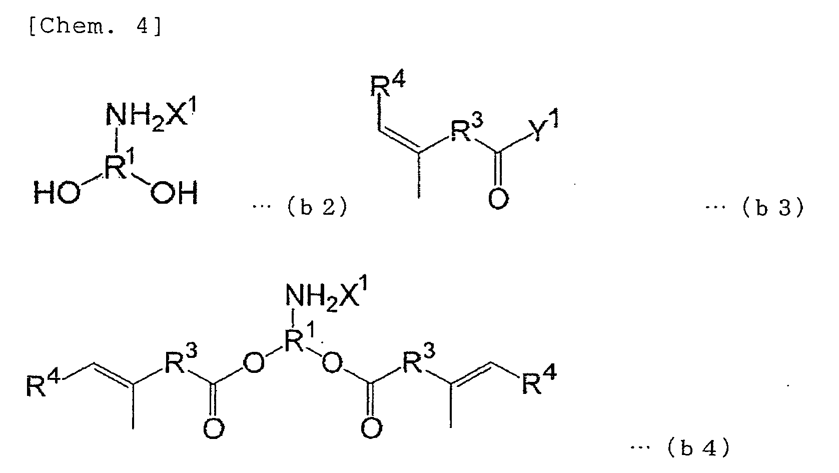
wherein R¹ is a C1-10, linear or branched saturated aliphatic group, X¹ is a mineral acid, each R³ is independently a C0-5, linear or branched alkylene group, each R⁴ is independently a hydrogen atom, a C1-6, linear or branched alkyl group, or an aryl group, and Y¹ is a hydroxyl group, a chlorine atom or R⁶O- (wherein R⁶ is a C1-6 alkyl group); 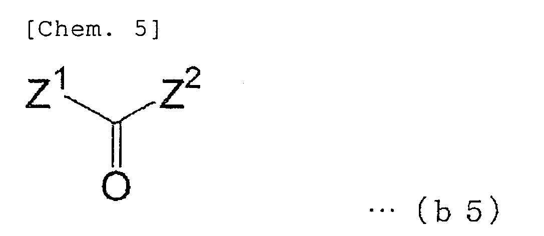
wherein Z¹ and Z² are each independently a chlorine atom, a bromine atom, R⁷O- (wherein R⁷ is a C1-6, linear or branched alkyl group, a C1-6, linear or branched alkenyl group or an optionally substituted aryl group), an imidazole residue or a pyrazole residue; 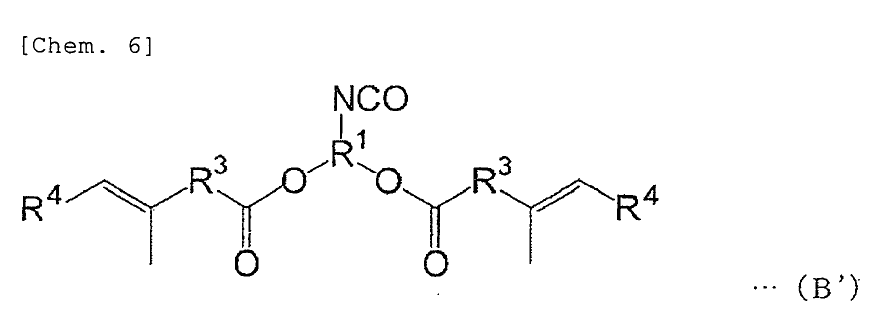
wherein R¹, R³ and R⁴ are the same as above.

14. The process for producing isocyanate group-containing, ethylenically unsaturated carboxylate compounds according to any one of claims 11 to 13, wherein the step of controlling the oxygen gas (O₂) concentration in a liquid phase to not less than 0.5 mg/L comprises:
a step of measuring the oxygen gas (O₂) concentration in the liquid phase;
a step of controlling the supply amount of oxygen gas (O₂) by controlling the introduction of a gas containing oxygen gas (O₂), based on the result of the measurement; and
a step of introducing the gas containing oxygen gas (O₂) to the liquid phase.
